# EUROPEAN PATENT SPECIFICATION

(11) **EP 0 493 446 B1**
(45) Date of publication and mention of the grant of the patent: **29.01.1997**
(21) Application number: 90913991.7
(22) Date of filing: 14.09.1990
(51) Int. Cl.: A61K 39/395, C07H 15/12, C07K 7/00, C07K 17/00, C07K 14/00

(54) **TREATMENT OF AUTOIMMUNE DISEASE**
BEHANDLUNG VON EINER AUTOIMMUN-KRANKHEIT
TRAITEMENT DE MALADIES AUTO-IMMUNITAIRES

(30) Priority: 15.09.1989 US 408123
(43) Date of publication of application: 08.07.1992
(60) Divisional application: 96101229.1
(73) Proprietor: TANOX BIOSYSTEMS, INC., Houston, TX 77025 (US)
(72) Inventor: CHANG, Tse, Wen, Houston, TX 77005 (US)
(74) Representative: Klunker . Schmitt-Nilson . Hirsch
(86) International application number: US9005229
(87) International publication number: WO9104055

(56) References cited:
- WO-A-89/06138
- DD-A- 0 230 879
- US-A- 4 545 986
- US-A- 4 661 586
- US-A- 4 744 983
- US-A- 4 816 567
- US-A- 4 894 443
- US-A- 4 925 787
- JPN. ARCH. INTERN. MED., Vol. 28(9), issued 1981, IWAMOTO "Antibody Mediated suppression of Antibody". Pg. 281-293 See entire Abstract No 82: 237819.
- Protein Engineering Vol 1(6) issued 1987, WHITTLE et al "Expression in Cos Cells of a mouse-Human Chimeric" B72.3 Antibody, page 499-506. See Abstract No. 88: 267403.
- Science, Vol 238, issued September 1985, ROGERS et al "Transfectomas Provide Novel Chimeric Antibodies", pages 1202-1207. See Abstract.
- Cell, Vol. 20 issued June 1980, ROGERS et al, "Two MRNAS with Different 3' Ends Encode membrane Bound and Secreted Forms of IgM Chain". Pages 303-312. See Fig 6 and Abstract.
- Int. J. Cancer, Vol. 43 (2) issued 1989 VAN D. J. KETAL, "Induction of Tumors
- Lysis by Bispecific MAb Recognizing Renal Cell carcinoma and CD3 Antigen" page 344-349. See Abstract No. 89: 183579.
- Rabbits et al., Nucleic Acids Research 9 (1981), 4509-4524

## Description

### Background

Autoimmune diseases encompass a wide variety of disorders, including: inflammatory central nervous system diseases such as multiple sclerosis and perivenous encephalomyelitis as well as certain neuropathies such as inflammatory demyelinating polyradiculoneuropathy; certain cardiovascular diseases such as rheumatic fever and some forms of myocarditis such as Chagas' disease; autoimmune cutaneous diseases such as pemphigus vulgaris and bullous pemphigoid; systemic lupus erythematosus (SLE); scleroderma; autoimmune hemolytic anemia; idiopathic thrombocytopenic purpura; autoimmune neutropenias; sperm and testicular autoimmunity; autoimmune diseases of the skeletal muscles such as myasthenia gravis; dermatomyositis and polymyositis; Grave's disease; thyroiditis and autoimmune endocrinopathies such as autoimmune Addison's disease, and Type I and Type II autoimmune polyglandular syndrome; pernicious anemia; insulin dependent diabetes; autoimmune liver diseases such as chronic active hepatitis and primary biliary cirrhosis; autoimmune kidney diseases such as glomerulonephritis, tubulointerstitial nephritis, anti-GBM nephritis, and anti-tubular basement membrane disease; autoimmune ocular diseases such as phycoantigenic uveitis, sympathetic ophthalmia, ocular cicatricial pemphigoid, Mooren's ulcer, scleritis, and anterior and posterior uveitis; mixed connective tissue disease; rheumatoid arthritis; some inflammatory bowel diseases; Sjorgren's syndrome; cryoblobulinaemia. All autoimmune diseases are caused by abnormal immune responses in the affected individuals against autologous antigens. The autoimmune antibodies bind to autologous antigens in the body fluids or on the surface of cells. This leads to a broad range of immune mechanisms resulting in tissue damages and alteration in cell functions, and to various manifestations of autoimmune diseases.

There are no effective treatments for the majority of the autoimmune diseases. The available treatments offer pain relief, or involve use of anti-inflammatory drugs which decrease the disease symptoms. Immunosuppressive drugs, such as cytocidal steroids, are also used to suppress the overall activity of the immune system.

Recently, monoclonal antibodies specific for surface antigens of T lymphocytes (T cells) or active immunocytes have been examined in animal model systems and in human clinical trials as therapeutic agents for autoimmune diseases. Some examples are monoclonal antibodies against (1) CD4 antigen on helper T cells, Wofsy, D. and Seaman, W.E., J. Exp. Med. 161:378 (1985); Waldor, M.K, *et al.* Science 227:415 (1985); Shizuru, J. A *et al*. Science 240:659 (1987); (2) Ia antigen on macrophages/monocytes, B cells and active T cells, Steinman, L. et al. Proc. Natl. Acad. Sci. U.S.A. 78:7111 (1981); Sriram, S. and Steinman, L. J. Exp. Med. 158:1362 (1983); McDevitt, H.O. *et al*. Ciba Found. Symp. 129:84 (1987) and; (3) Interleukin-2 receptors on activated T cells, Kelley, V.E. *et al.* J. Immunol, 140:59 (1988); Strom, T.B. *et al.* Pro. Clin. Biol. Res. 224:227 (1986). These monoclonal antibodies have been shown to be able to destroy or down-regulate *in vivo* lymphocytes bearing the surface antigens with which the antibodies react. The immune mechanisms leading to the specific target cell depletion are believed to include antibody-dependent cellular cytotoxicity (ADCC), complement-dependent cytolysis, and probably some other mechanisms. The antibodies specific for surface antigens of T cells or activated immunocytes can suppress the entire immune systems and, therefore, the antibody responses against the autologous antigens. This general immunosuppression alleviates the severity of the autoimmune diseases in patients.

General immunosuppression resulting from the depletion of T cells or activated T cells, however, can be a therapy with side-effects so deleterious that they outweigh the benefits for many patients. T cells play central roles in the development, maturation, and regulation of various branches of the immune system, including cytotoxic T cells, antibody response, and phagocytic mononuclear and polymorphonuclear cells. It is, therefore, desirable to develop a therapy that will down-regulate or selectively deplete the antibodies associated with autoimmune disease.

For example, in patients with rheumatoid arthritis, some species of IgG have abnormal epitopes that induce the production of IgG and IgM, known as rheumatoid factors. These autoimmune IgG bind to the rheumatoid factors, forming immune complexes, leading to the pathogenesis of rheumatoid arthritis diseases. In many other autoimmune diseases, production of immunoglobulin of IgG and IgM isotypes is stimulated, and in some autoimmune diseases, the production of other isotypes may be stimulated. It would be very desirable to selectively suppress the synthesis of the isotypes associated with particular autoimmune diseases, and in particular, the isotypes IgG and IgM which are most frequently associated with such diseases.

### Summary of the Invention

The invention relates to treating autoimmune diseases by selectively suppressing or depleting B-cells which produce immunoglobulin of the isotypic class associated with an autoimmune disease, or by selectively suppressing or depleting precursors of these cells. The inventors could determine the amino acid sequence of the extra cellular epitope of the B-cell membrane bound form of IgM or IgG which is not present on the secreted form of the immunoglobulins, and have then realised that diseases can be treated by targeting of these epitopes which are expressed on the B-cell surface by antibodies and related products.

The preferred embodiment includes treating autoimmune diseases through targeting of epitopes unique to the form of IgG and IgM isotypes which are expressed on the B cell surface, and the antibodies and related products which can be used for targeting of these epitopes. In another embodiment of the invention, the B cells which express and secrete antibody of a particular isotype are eliminated or suppressed by administering a product which stimulates an isotype-specific immune response to the B cell (for example, production of antibody that binds specifically to the B-cell).

The human immunoglobulin isotypes are IgG (having four subclasses), IgA (having two subclasses), IgM, IgD, and IgE. IgG causes opsonization and cellular cytotoxicity and crosses the placenta, IgA functions on the mucosal surface, IgM is most effective in complement fixation, and IgE mediates degranulation of mast cells and basophils. The function of IgD is still not well understood. The heavy chains of IgG, IgA, IgM, IgD and IgE are respectively designated the γ, α, µ, δ, and ε chains. The cell-bound forms of each of these immunoglobulin isotypes are expressed on the B cell surface, and the B cells which produce a secreted, soluble form of that isotype also express the cell-bound form of the particular isotype.

Extending from the C termini of the heavy chains of the cell-bound forms of the immunoglobulins are membrane anchoring peptides. These membrane anchoring peptides span the cell membrane lipid bilayer, thereby affixing the associated immunoglobulin to the cell membrane surface. The extracellular segments of these peptides are unique for different isotypes, but tend to be very similar among different subclasses of a particular isotype. The extracellular segment of each immunoglobulin isotype forms, in whole or in part, epitopes unique to the B cells which express that isotype. These membrane-bound immunoglobulin isotype-specific ("*migis*") extracellular epitopes are not present on the secreted, soluble form of the immunoglobulins, which are not bound to the cell surface by the membrane anchoring peptides.

The antibodies and other related products of the invention bind to the *migis* epitopes, and preferably the *migis-µ* or *migis-γ* epitopes, which are present on the B cell surface. The B cells which produce the particular isotype, *e.g.*, IgG or IgM, can then be eliminated or controlled by a number of immune mechanisms.

Resting B cells which are immunocompetent but not yet activated express IgM and IgD. Once activated and committed to secrete antibodies, these B cells can express any of the five isotypes. Accordingly, a product which targets the *migis-*µ epitope will deplete or suppress resting, immunocompetent B cells, and, as a result, will cause depletion or suppression of B cells producing all five of the immunoglobulin isotypes. However, the immunosuppression is limited to antibody production, not affecting the functions of T cells and macrophages and granulocytes.

The antibodies of the invention include both polyclonal and monoclonal antibodies. To avoid or minimize an immunogenic reaction to the antibodies administered, several related products including chimeric antibodies (which are a combination of an animal variable binding region and a human constant region), single chain antibodies, human antibodies, or fragments of human or chimeric antibodies, can be used. Antibodies in which the entire constant portion and most of the variable region is human derived, and only the antigen binding site is animal derived, can also be used. Several other derivative products of the antibodies of the invention, described further below, can also be used in therapy.

The invention will now be further described with reference to the drawings.

### Brief Description of the Drawings

Fig. 1A shows the genomic DNA sequence of a segment of human γ-1 chain, the sequence of a first membrane exon which is proposed to encode for the membrane anchoring peptide being underlined, and the flanking nucleotide sequences not being underlined.

Fig. 1B shows the genomic DNA sequence of a segment of human γ-1 chain, the sequence of a second membrane exon which is proposed to encode for the membrane anchoring peptide being underlined, and the flanking nucleotide sequences not being underlined.

Fig. 2A shows the nucleotide sequences of the two membrane exons of human γ-1 chain shown in Figs. 1A, 1B (some of the flanking sequences are shown by lower case letters), as well as the corresponding nucleotide sequences for human γ-2, γ-3, and γ-4 chains.

Fig. 2B shows the deduced amino acid sequences encoded for by the membrane exons shown in Fig. 1A, the proposed extracellular segment being indicated.

Fig. 3A shows the sequence of a segment of cDNA of human γ-1 chain from mRNA of IM9 cells, linking the CH3 domain to the membrane exon shown in Fig. 1A, to the membrane exon shown in Fig. 1B, to the 3' untranslated region.

Fig. 3B shows the splicing donor/acceptor sites of the segment of cDNA shown in Fig. 3A.

### Detailed Description of the Preferred Embodiments and Their Manner and Process of Making and Using

Membrane anchoring peptides are parts of the heavy chains of the associated isotypes and anchor the isotypes to the cell surface. For IgM and IgG respectively, these anchoring peptides have lengths of 41 and 71 amino acids. These membrane anchoring peptides can be divided into three segments, each segment located differently with respect to the plasma membrane. The middle segments of 25 uncharged and hydrophobic residues are in the membrane lipid bilayer. The C-terminal hydrophilic segments of the membrane anchoring peptide of IgM has 3 residues, while the corresponding C-terminal hydrophilic segment of IgG has 28 residues. These C-terminal segments are proposed to be intracellular. The segment of IgM towards the N-terminus contains 13 amino acid residues, whereas the corresponding segment of IgG contains 18 residues. These segments towards the N-terminus are highly acidic and hydrophilic and are therefore proposed to be on the extracellular surface of the plasma membrane. Because the N-terminus segments are probably extracellular, they are likely to be exposed and accessible to antibodies and can be targeted, thereby providing a means of destroying B cells producing a particular isotype. These extracellular segments form, in whole or in part, epitopes designated herein as the *migis* epitopes.

The proposed sequences of the membrane anchoring peptides of IgG (subclasses 1 to 4) were determined by the methods outlined below. The sequences of the membrane anchoring peptides of IgD and IgM was previously published, although, as discussed below, the published sequence of human IgM was shown to be partially incorrect. The sequence of the *migis* segment of IgA appears in international application No. PCT/US90/03532, and the sequence of one isoform of the *migis* segment of IgE appears in published international application No. PCT/US88/04706. The sequence of a proposed *migis* segment of a second isoform of IgE is:
GLAGG·SAQSQ·RAPDR·VLCHS·GQQQG·LPRAA·GGSVP·HPRCH· CGAGR·ADWPG·PPELD·VCVEE·AEGEA·PW.
The sequence of a proposed *migis* segment of a third isoform of IgE is:
GAAVP·LSHAA·GEAPD·LPRLH·QRPPA·PRLGR·GHSRS·TRPSF·FSASA.

Peptides containing the extracellular *migis* epitopes, or segments or immunologic equivalents of these peptides, can be used as immunogens. Such immunogenic peptides can be synthesized by conventional techniques, such as with the RaMP system (DuPont DeNemours & Co.), which applies Fmoc chemistry. Alternatively, recombinant peptides or immunoglobulin heavy chains (or portions thereof) containing the *migis* epitopes may be biosynthesized by expressing in *E. coli* or eukaryotic cells the gene segments containing the coding sequence of these peptides.

When using a synthetic peptide segment as an immunogen, it is usually more effective to conjugate it to a protein carrier, for example, hepatitis B surface antigen, core antigen, or preferably keyhole lympit hemocyanin (KLH). If the peptidic segment lacks a lysine residue or if the lysine residue is in the middle part of the segment, it is desirable to add a lysine residue at the C-terminal end. Because the N-terminus already has an α-amino group, the modified synthetic peptidic will have two available amino groups for linking.

Multiple molecules of peptides can be conjugated to each molecule of the carrier protein. With KLH, a preferred molar ratio for peptide/KLH is 10. The method of conjugation is very well established. Cross-linkers such as glutaraldehyde or bis (sulfosuccinimidyl) suberate or preferably disulfosuccinimidyl tartrate (Catalogue #21579, 20591, Pierce Chemical Co., Rockford, IL) can be used.

As immunogens, these peptides can be used to make monoclonal antibodies which are specific for them, using the protocol described further below. Monoclonal antibodies to the *migis* epitope of human ε chain have been made, using essentially the same protocol described below. See International Application No. PCT/US88/04706. The success in making monoclonal antibodies to an epitope on the ε chain *migis* segment, which is 15 amino acids in length, indicates that one should also be able to also make such antibodies to the *migis-*µ or *migis*-γ segments, the former being of comparable length and the latter being longer than the *migis*-ε segment. It is also noted that the production of antibodies against this segment of human δ chain (IgD) has been reported. See Blattner, F.R*. et al*, Nature, 307:417-422 at 418 (1984).

The immunogenic peptides of the invention can also be used to immunize rabbits, goats, rats, or mice (or even another human being) to prepare polyclonal antibodies to the extracellular *migis* epitopes. Monoclonal antibodies that react with the peptides of the invention can be further screened for positive specific reactivity with cells bearing a specific isotype. The monoclonal antibodies can then be applied *in vivo.* Polyclonal antibodies made against peptides of the invention, however, generally contain almost entirely antibodies that react with the synthetic peptide but not the native molecules. Whether the polyclonal antibodies made against synthetic peptides can react with intact cells must be tested.

When preparing monoclonal antibodies, it is not necessary to use the synthetic or recombinant peptides in both immunization and antibody identification. For example, in immunizing mice for preparing spleen cells for fusion with myeloma cells, the immunogen may be the membrane-bound immunoglobulin isolated from the plasma membrane of immunoglobulin-bearing myeloma cells, such as IgG-expressing IM-9 cell line, or it may be the myeloma cells themselves. Transfectomas, which are developed by transfecting mouse myeloma cells with genes of human immunoglobulin heavy chains and light chains and which express on their cell surface membrane-bound immunoglobulins, may also be used as immunogens. For initial monoclonal antibody identification following immunization, the aforementioned synthetic peptides conjugated to ovalbumin or bovine serum albumin, which are not used as carrier proteins in immunization, are preferably used.

Lymphocytes from the spleen or lymph nodes of immune mice and rats can also be used to prepare hybridomas secreting monoclonal antibodies specific for the *migis* epitopes. A preferred protocol for preparing monoclonal antibodies is to fuse immune spleen cells of mice with non-secreting mouse myeloma cells, such as NS-1 or SP2/0 cells, using polyethylene glycol.

A preferred immunization protocol for preparing monoclonal antibodies is to inject into each mouse 50 µg of the conjugate of KLH and the recombinant or synthetic peptides of the invention in complete Fruend's adjuvant. Two and four weeks later, the same amount of antigen is given subcutaneously in incomplete Fruend's adjuvant. After about six weeks, the fourth antigen injection is given intraperitoneally in saline. Mice are sacrificed 4 days after the last injection and the spleens are removed for preparing single cell suspensions for fusion with myeloma cells.

A similar protocol can be used for immunization with purified native human membrane-bound immunoglobulins (having attached membrane anchoring peptide segments) isolated from the plasma membrane of immunoglobulin-bearing human myeloma cells, such as IM-9 cells. When human immunoglobulin-bearing cells are used as the immunogen, 1 x 10⁷ cells are injected intraperitoneally at two week intervals.

The fusion procedure with polyethylene glycol and other various procedures concerning cloning and hybridoma culturing have been well established. The preferred protocol is the well-known one described by Hudson, L. and Hay. F.C. (Practical Immunology, 2nd edition, pp. 303-313, 1980, Blackwell Publishing Co., Boston).

The screening of hybridomas for monoclonal antibodies (or the identification of polyclonal antibodies) reactive with the *migis* epitopes of the invention can be performed with an enzyme linked immunosorbent assay (ELISA) using the synthetic peptide as the solid phase antigen. A preferred solid phase antigen is the conjugate of a membrane anchoring peptide with a carrier protein different from that used in the immunogen, such as bovine serum albumin or ovalbumin. Monoclonal antibodies specific for the *migis* peptide of the IgG or IgM isotype are then screened for specific binding to B cell lines and B cells expressing the particular isotypes by using immunofluorescence flow cytometric analyses.

Generally, the *migis* epitope-specific monoclonal antibodies which are first obtained will be murine-derived, and thus may be immunogenic or allergenic in human therapy. It is therefore desirable to produce chimeric antibodies (having an animal variable region and a human constant region), or to use human immunoglobulin expression libraries (Stratagene Corp., La Jolla, California) to produce fragments of human antibodies (V_{H}, V_{L}, F_{V}, Fd, Fab, or F(ab')₂) and then construct whole human antibodies using techniques similar to those for producing chimeric antibodies. In addition, one can create antibodies in which the entire constant portion and most of the variable region are human-derived, and, only the antigen binding site is mammalian derived. See Riechmann, L. *et al.*, Nature 332:323-327 (1988). Further, one can create single peptide chain antibodies in which the heavy and light chain Fᵥ regions are connected. See Huston, J.S. *et al.*, Proc. Natl. Acad. Sci. USA 85:5879-5883 (1983). All of the wholly and partially human antibodies are less immunogenic than mammalian equivalents, and the fragments and single chain antibodies are less immunogenic than whole antibodies. All these types of antibodies are therefore less likely to evoke an immune or allergic response. It is noted that an immune response could reduce the effects of the antibodies which are administered before such antibodies could function to suppress or deplete the IgG or IgM expressing B cells.

Monoclonal antibodies specific for the extracellular *migis* epitopes of IgM or IgG isotype can be used to reduce or eliminate the B cells expressing these isotypes by ADCC, complement-mediated cytolysis, or other cytolytic or regulatory immune mechanisms. For example, antibodies of certain IgG subclasses, such as mouse IgG₂ₐ and human IgG₁ and IgG₃, can mediate ADCC carried out by certain Fc receptor-bearing phagocytic leukocytes. Administration of such mouse IgG₂ₐ antibodies, chimeric antibodies bearing human γ-1 or γ-3 chains, or human IgG₁ or IgG₃ antibodies can be used to down-regulate or lyse B cells of a particular isotype. These antibodies can be administered to suppress the immune system because they cause lysis of a substantial portion of the B cells which express immunoglobulin of the targeted isotypic class, and consequently, they will reduce substantially the production of the immunoglobulin isotype. The monoclonal antibodies of the invention can also be used as targeting agents for cytotoxic cells.

The monoclonal antibodies of the invention can also be used as carrier agents of or cytotoxic drugs for delivering an effector substance, by conjugating the monoclonal antibodies to these substances. A toxin-antibody conjugate will bind and directly kill B cells producing the isotypes IgM or IgG. These toxins are cytolytic or cytotoxic agents, including cytotoxic steroids, gelonin, abrin, ricin, *Pseudomonas* toxin, diphtheria toxin, pokeweed antiviral peptide, tricathecums, radioactive nuclides, and membrane-lytic enzymes (such as phospholipase). The antibody and the agent can be conjugated by chemical or by genetic engineering techniques. The toxin-antibody conjugates may be used alone or in combination with the free antibodies of the invention.

The antibodies of the invention (and the toxin conjugates, fragments, and other derivatives) are administered systemically, and preferably intravenously. They can be administered in any pharmaceutically acceptable vehicle. More than one antibody against one isotype can be administered at the same time. For example, different combinations of anti-*migis*-µ and anti-*migis*-γ antibodies can be used simultaneously to achieve immunosuppression.

Another therapeutic alternative involves active immunization, wherein antibodies specific to the *migis* epitopes (preferably the *migis*-µ or *migis*-γ epitopes) are endogenously produced *in vivo.* These endogenously produced antibodies bind the *migis* epitopes and cause destruction of the associated B cells. Production of such antibodies can be induced either by administering an immunogenic *migis*-bearing membrane anchoring peptide of the invention, or a paratope-specific, anti-idiotypic antibody. Anti-idiotype antibodies against the paratope of the antibodies of the invention conformationally resemble the extracellular *migis* epitopes. These anti-idiotypic antibodies can be used to actively immunize against the *migis* epitopes and induce the endogenous formation of antibodies against the *migis* epitopes. Such paratope-specific, anti-idiotypic antibodies are administered to a patient in an immunogenic amount sufficient to induce the formation of antibodies against B cells expressing the targeted isotype. These anti-idiotypic antibodies are preferably administered as chimeric antibodies or human antibodies, to minimize any immune response against them. They may also be any of the antibody fragments, V_{H}, V_{L}, F_{V}, Fd, Fab, or F(ab')₂ (which also may be chimeric or human in nature).

Certain factors, such as granulocyte monocyte-colony stimulation factor (GM-CSF) or monocyte-colony stimulation factor (M-CSF), are known to induce the proliferation of leukocytes, including those mediating ADCC. In *in vitro* experiments, GM-CSF and M-CSF have been shown to augment the ADCC activity on tumor cells mediated by monoclonal antibodies specific for surface antigens expressed on the tumor cells. The therapeutic effect of specific monoclonal antibodies of the invention, conjugates, or polyclonal antibodies in suppressing the immune response could perhaps be enhanced by combining them with factors that augment ADCC activities.

Derivative antibodies can be made which draw cytotoxic cells such as macrophages or cytotoxic T cells toward the targeted immunoglobulin-expressing B cells. These derivative antibodies include bi-specific antibodies having a specificity for a receptor of a cytotoxic cell and a specificity for the targeted Ig expressing B cells. Such hybrid bi-specific antibodies can include two different Fab moieties, one Fab moiety having antigen specificity for the targeted *migis* epitopes (preferably the µ or γ chain *migis* epitopes) and the other Fab moiety having antigen specificity for a surface antigen of a cytotoxic cell, such as CD3 or CD8. The bi-specific antibodies of the invention can be a single antibody having two specificities, or a heteroaggregate of two or more antibodies or antibody fragments. See, e.g., C. Reading, U.S. Patent Nos. 4,474,893 and 4,714,681; Segal *et al,* U.S. Patent No. 4,676,980.

While monoclonal antibodies of the invention can be used for *in vivo* applications, they may also be used in extra-corporeal *ex-vivo* applications. The IgM or IgG bearing B cells in the circulation of the patients can be removed by an affinity matrix (antibody immobilized on a solid phase) which is conjugated with the monoclonal antibodies of the invention.

Another use for the antibodies of the invention is for determining numbers and relative proportions of B lymphocytes bearing particular isotypes in mixed leukocyte populations. The *migis* specific antibodies will not react with cells which bear secreted immunoglobulins via such cells' Fc receptors. Such cells include macrophages and activated T cells. The profile of the B cells may indicate the immune status of the individual, and whether further suppression is desirable. The same information can also indicate how much antibody is needed to deplete a substantial portion of B cells bearing a particular isotype. For this purpose, antibodies can be used in standard assays which are used to determine cell surface antigens. In general, the antibodies are contacted with a sample of the leukocytes to be tested under conditions which allow the antibodies to bind isotype-bearing cells in the sample. The cells are then examined for binding of antibody. This can be accomplished by conventional cell staining procedures, for example, a fluorescently labeled second antibody can be used to detect binding of antibody.

### A. Sequence of the Migis Segment of Human IgM

The sequence of the *migis* segment of human IgM was previously published by Rabbits, T.H. *et al.* (1981) Nucl. Acids Res. 9:4509-4524, who indicated that it was the same as the corresponding segment of mouse IgM, which is as follows:
Mouse IgM EGEVN·AEEEG·FEN
However, using the cDNA sequencing described below, the sequence of the extracellular *migis* segment of human IgM was deduced to actually be:
Human IgM EGEVS·ADEEG·FEN
The features and properties of the various segments of the whole human IgM membrane anchoring peptide is shown below in Table I.

**TABLE I**

| | First segment | Middle segment | Last segment | |
|---|---|---|---|---|
| Properties: | Hydropholic Highly Acidic | Hydrophobic No charged residues | Hydrophilic | |
| Physical Location: | On exterior surface | In membrane lipid bilayer | On cytoplasmic surface | Totals |
| Human IgM* | 13 | 25 | 3 | 41 |

| | | | | |
|---|---|---|---|---|
| *The numbers represent the number of amino acid residues. | | | | |

The nucleotide sequence in the membrane region of human µ mRNA was determined by the following procedure. Total mRNA was isolated from peripheral blood mononuclear cells of a normal donor, and from a human surface IgM-expressing cell line, RPMI-1788 (obtained from the ATCC, Rockville, Maryland) according to the techniques described in Molecular Cloning, (Sambrook, Fritch, and Maniatis, Cold Spring Harbor, 1989). Two oligonucleotides were synthesized and used as the primers for PCR, based on the known DNA sequences published by Rabbitts, T.H. *et al,* Nucleic Acids Research 9: 4509-4514 (1981). These primers had the sequences:
5' end: 5'CCAACAGGGTCACCGAGAG3'
3' end: 5'CCTTGAACAAGGTGACGGT3' (complementary).
The amplified DNA products were cloned and the sequences of the gene segments for both the normal donor and the RMPI-1788 cells were determined to be:
AG·GGG·GAG·GTG·AGC·GCC·GAC·GAG·GAG·GGC·TTT· GAG·AAC. From this sequence, the amino acid sequence of the extracellular *migis*-µ segment (shown above) was deduced. The conditions and temperatures of the PCR were similar to those used in sequencing the *migis*-γ segments, discussed in Section B (ii) below.

### B. Membrane Anchoring Peptides of Human γ Chain

### (i) Extracellular Migis-γ Segments

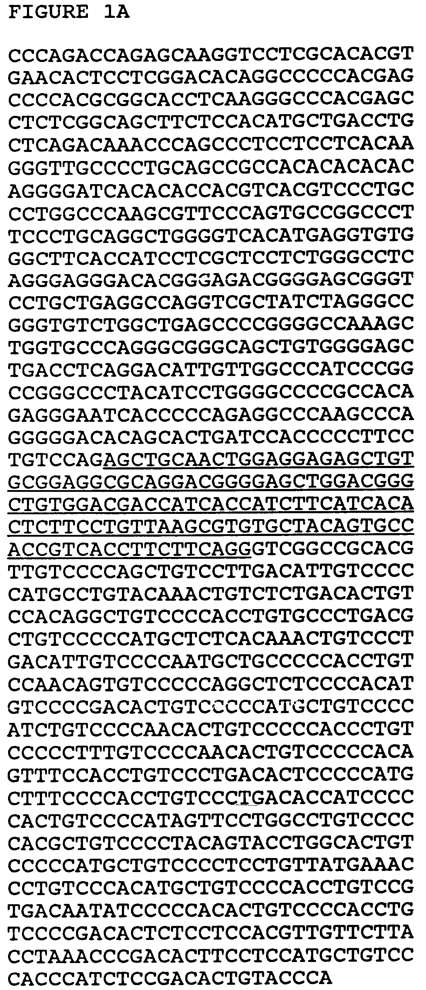

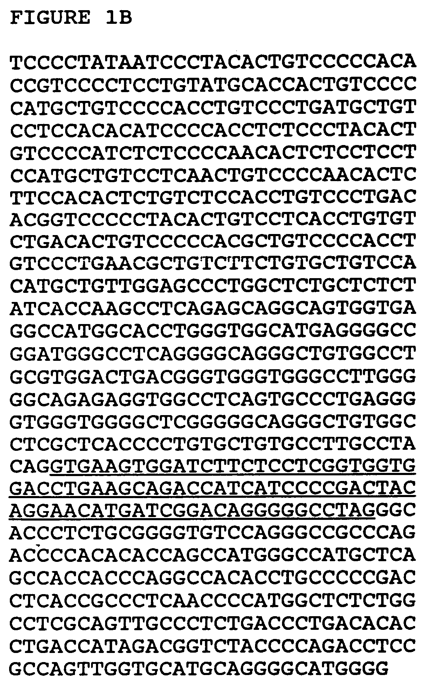

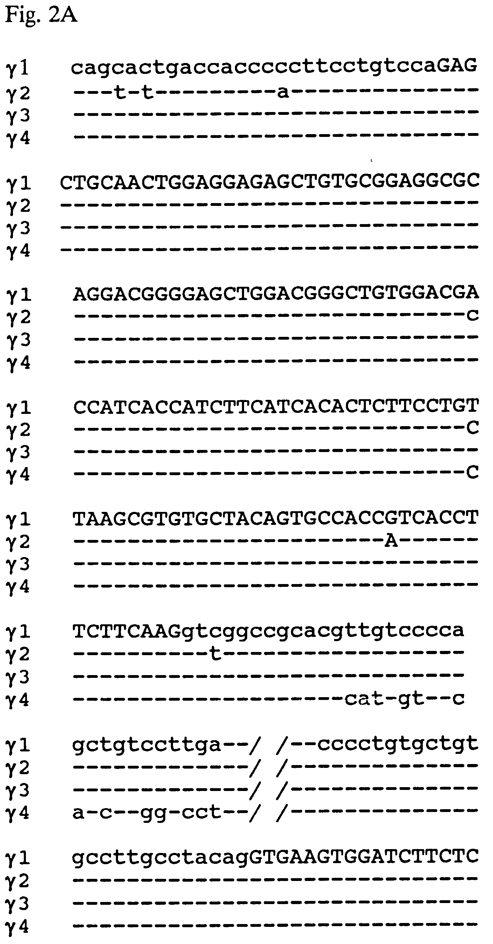

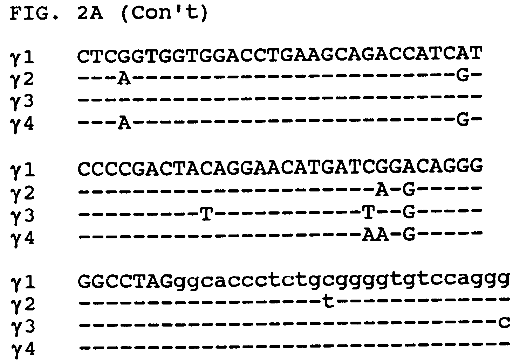

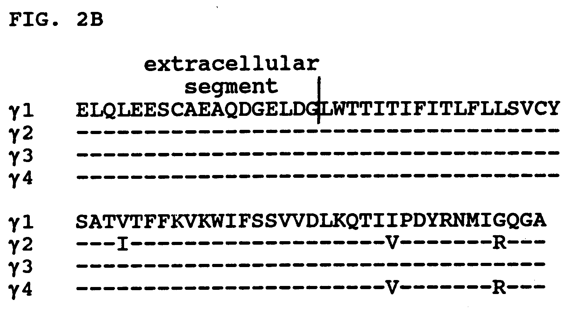

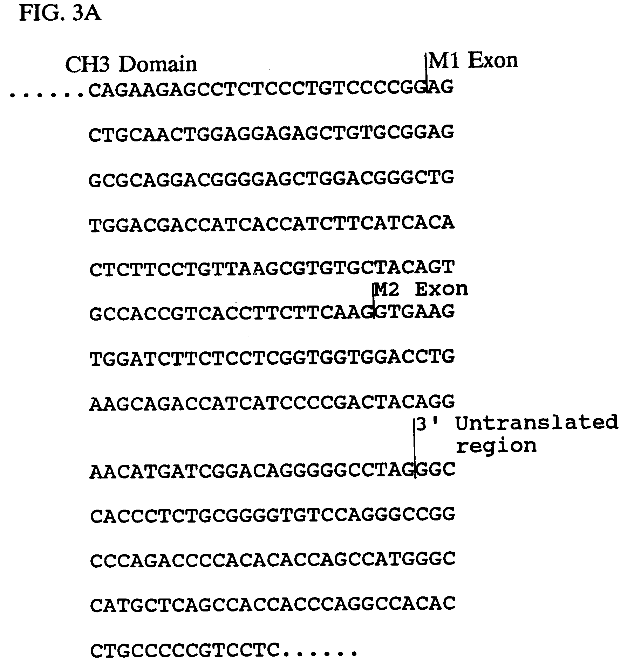

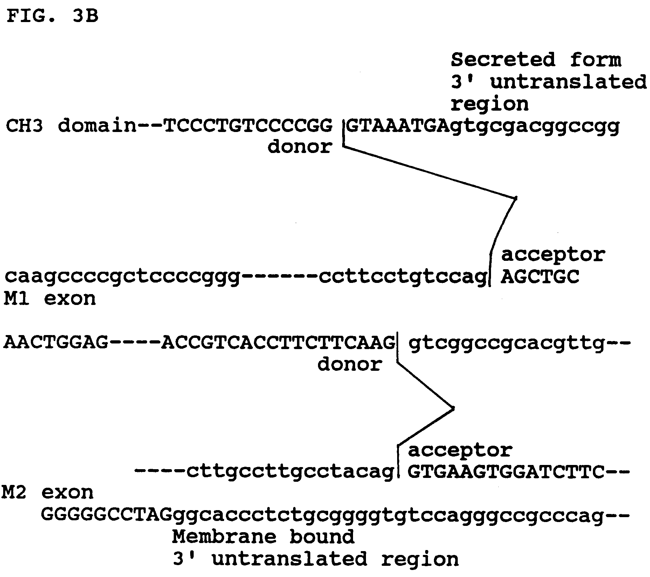

The genomic DNA sequences of the membrane anchoring segments of human γ-1, γ-2, γ-3, γ-4, were determined by the methods described below. Two membrane exons encode for these DNA sequences. In Figs. 1A, 1B, respectively, the genomic nucleotide sequences corresponding to the two membrane exons of human γ-1 are underlined. The upper case letters in Fig. 2A denote the genomic DNA sequence of these two membrane exons for human γ-1, γ-2, γ-3, and γ-4, the flanking nucleotide sequences being indicated by lower case letters. Fig. 2B shows the deduced amino acid sequences encoded by the exons of Fig. 2A. Fig. 3A shows the DNA sequence of a gene segment of human γ-1 chain which includes the two membrane exons and some flanking sequences. Fig. 3B depicts the splicing donor sites and acceptor sites involved in the formation of the segment shown in Fig. 3A.

Based on the genomic DNA sequences, mRNA sequences, and the identified splicing mechanisms, the predicted amino acid sequences of the membrane anchoring peptides of human γ-1, γ-2, γ-3, and γ-4 shown in Fig. 2B were determined. By comparing to the sequences of membrane anchoring peptides of other known immunoglobulins, the hydrophobic stretch that presumably spans through the membrane lipid bilayer can be identified. This sequence of 25 amino acid residues is LWTTI· TIFIT· LFLLS· VCYSA· TVTFF. To the N terminal end of this hydrophobic stretch is the segment proposed to form the *migis* epitope. Thus, for all human γ chains, the sequence of 18 amino acid residues (as shown in Fig. 2B) which form the extracellular *migis* epitope, entirely or in part, is:
ELQLE·ESCAE·AQDGE·LDG.

These proposed *migis* segments are believed to be extracellular and accessible by antibodies, based on the fact that they have multiple acidic residues, which suggests that they are hydrophilic. Since the *migis* segments for human γ-1, γ-2, γ-3, and γ-4 are identical, it is likely that a monoclonal antibody can be prepared which recognizes cell-bound but not secreted IgG of any of the four subclasses.

### (ii) Sequencing the Human migis-γ Segment

The proposed sequence of the membrane anchoring peptides of human γ chains was determined by the methods set forth below. The genomic DNA clones containing γ-1, γ-2, γ-3, and γ-4 could be obtained from a genomic DNA library, such as λ·FIX phage library of genomic DNA of human lung fibroblast line, WI38, provided by Strategene (LaJolla, California). The human genomic γ-1, γ-2, γ-3, and γ-4 DNA segments, which were originally provided by Dr. Sherrie Morrison (previously of Columbia University and now of the University of California, Los Angeles), were used. The human B cell line, IM9, expressing IgG₁ on the surface was obtained from the American Type Culture Collection, Rockville, Maryland. Peripheral blood mononuclear cells from normal blood donors were used as a source of mRNA for examining human γ chain sequences. The various restriction enzymes used were from Boehringer Mannheim, New England Biolabs and Bethesda Research Laboratories. The Erase-a-base kit, which can be used to construct nested deletions, was obtained from Promega Corp. Dideoxysequencing of double stranded templates was performed using T7 sequencing kit from Pharmacia/LKB. The Bluescript vector was obtained from Strategene Cloning Systems. All clones were DH5αF' cells (Bethesda Research Laboratory).

To facilitate mapping and sequencing, the genomic γ clones were subcloned into Bluescript SK II+. In order to locate the membrane regions, a set of the nested deletions was created using the Erase-a-base kit. The γ plasmid was cut with SacI and NcoI. SacI gives 5' recessed ends which are protected from Exo III digestion.

Several µg were digested with Exo III and aliquots were withdrawn at 30 second intervals. These aliquots were placed in S1 digestion mix, which stops the Exo III reaction. After 30 minutes, the S1 reaction was stopped by heating to 70°C. Following a brief treatment with Klenow fragment, each aliquot was recircularized with T4 DNA ligase. Plasmids with deletions of various lengths were sequenced and compared to mouse γ chain membrane regions. After identifying clones which contain a part of the membrane regions, oligonucleotide primers were constructed to complete the sequencing of segments covering the membrane exons.

For sequencing cDNA, the cDNA clones were prepared from cDNA synthesized from RNA isolated from IM9 cells or peripheral blood mononuclear cells from normal donors, by using a primer in the 3'untranslated region 5'GTTGAGGGCGGTGAGACG3' (complementary sequence from genomic DNA) and AMV reverse transcriptase. The RNA isolation and cDNA preparation were performed according to Molecular Cloning (Sambrook, Fritch, and Maniatis, Cold Spring Harbor, 1989). Second strand synthesis and amplification of desirable gene segments was carried out using PCR with a 3' primer, 5'GGCAACTGCGAGGCCAGAG3' from the 3' untranslated region and a primer with the sequence from the CH3 domain (5'AGAAGAGCCTCTCCCTGTC3'). The PCR conditions were as follows: denaturation, 94°C, 1 min; annealing, 60°C, 2 min; polymerase reaction, 72°C, 3.5 min; 35 cycles.

Two bands of 350 and 500 nucleotide were isolated from IM9 RNA source. These were cloned into Bluescript SK II+. Upon sequencing, clones with 350 bp inserts having γ-1 sequences were identified. This cDNA was labeled with ³²P and used to screen by colony hybridization a number of cDNA clones derived from the PCR product of RNA from normal lymphocytes. The inserts of the positive clones were prepared and the nucleotide sequences determined.

## Claims

1. A DNA sequence that encodes the extracellular epitope of the B cell membrane-bound form of IgM or IgG, said epitope not being present on the secreted form of said immunoglobulins said DNA sequence comprising one of the sequences represented by upper case letters in Figure 2A, or
AG.GGG.GAG.GTG.AGC.GCC.GAC.GAG.GAG.GGC.TTT.GAG.AAC.

2. A peptide containing the extracellular epitope of the B cell membrane-bound form of IgM or IgG, said epitope not being present on the secreted form of said immunoglobulins and comprising one of the following amino acid sequences:
EGEVS.ADEEG.FEN; or
ELQLE.ESCAE.AQDGE.LDG.

3. The peptide of claim 2 conjugated to a substance which increases the immunogenicity of the peptide.

4. The peptide of claim 3, wherein the substance is hepatitis B surface antigen, core antigen, or keyhole limpet hemocyanin.

5. Antibody which specifically binds to the extracellular epitope of the B cell membrane-bound form of IgM or IgG, said epitope not being present on the secreted form of said immunoglobulins.

6. The antibody of claim 5 which, upon binding, labels the B cells associated with the membrane-bound immunoglobulin for destruction.

7. The antibody of claim 5 or 6, wherein said antibody binds to an epitope on one of the following peptide segments:
EGEVS.ADEEG.FEN;
ELQLE.ESCAE.AQDGE.LDG.

8. The antibody of any one of claims 5 to 7, wherein said antibody is a monoclonal antibody.

9. The antibody of claim 8, wherein said monoclonal antibody is a human monoclonal antibody or a fragment thereof.

10. A fragment of the antibody of any one of claims 5 to 9, or a peptide containing the antigen binding portion of said fragment, said fragment or peptide having the binding specificity of said antibody.

11. The antibody of claim 9, wherein the human antibody fragment is selected from the group consisting of V_{H}, V_{L}, F_{V}, Fd, Fab, Fab', F(ab')₂, and protein or peptide analogues thereof.

12. The antibody of claim 8, wherein said monoclonal antibody is a chimeric antibody having animal variable regions and human constant regions.

13. The antibody of claim 12, wherein said chimeric antibody has murine variable regions and human constant regions.

14. The antibody of claim 12 or 13, wherein said chimeric antibody has the antigen binding portion of its variable region derived from an animal and other portions of its variable region, and its entire constant region, derived from a human.

15. The antibody of any one of claims 8 to 9 or 11 to 14, wherein said monoclonal antibody is one of the following isotypes and subclasses: murine IgG2a, human IgG1 and human IgG3.

16. A continuous, stable cell line which produces the monoclonal antibody of any one of claims 8 to 9 or 11 to 14 or the fragment of claim 10.

17. The cell line of claim 16 which is a hybridoma.

18. The cell line of claim 17 which is a transfectoma.

19. A monoclonal anti-idiotypic antibody specific for the paratope of the antibody of any one of claims 5 to 15.

20. The monoclonal anti-idiotypic antibody of claim 19 which is a chimeric antibody, having an antigen-binding region of animal origin and a human constant region or a fragment thereof.

21. The monoclonal anti-idiotypic antibody of claim 20, wherein the antibody fragment is selected from the group consisting of V_{H}, V_{L}, F_{V}, Fd, Fab, Fab', F(ab')₂, and protein or peptide analogues thereof.

22. A continuous stable cell line which secretes the monoclonal antibody of any one of claims 19 to 21.

23. A pharmaceutical composition comprising an antibody of any one of claims 5 to 15 or 19 to 21, or the peptide of any one of claims 2 to 4.

24. The pharmaceutical composition of claim 23 for treating an autoimmune disease associated with a particular immunoglobulin isotype.

25. The pharmaceutical composition of claim 24, wherein the immunoglobulin is IgG or IgM isotype.

26. The pharmaceutical composition of claim 24 or 25, wherein the autoimmune disease is rheumatoid arthritis, multiple sclerosis, myasthenia gravis or systemic lupus erythematosus.

27. Use of the antibody of any one of claims 5 to 15 or 19 to 21, or the peptide of any one of claims 2 to 4 for the preparation of a pharmaceutical composition for treating an autoimmune disease.

28. Use according to claim 27, wherein the autoimmune disease is rheumatoid arthritis, multiple sclerosis, myasthenia gravis or systemic lupus erythematosus.

29. Use of the antibody of any one of claims 5 to 15 or 19 to 21 for determining in a standard assay numbers and relative proportions of B lymphocytes bearing particular isotypes in mixed leukocyte populations.

30. Method for the production of a monoclonal antibody or a fragment thereof which comprises cultivating the cell line of claims 16 to 18 or 22 and isolating the monoclonal antibody or the fragment produced.

## Patentansprüche

1. DNA-Sequenz, die das extrazelluläre Epitop der B Zellen-Membran-gebundenen Form von IgM oder IgG codiert, welches Epitop an der ausgeschiedenen Form des Immunglobulins nicht vorhanden ist, wobei die DNA-Sequenz eine der in Fig. 2A durch Großbuchstaben dargestellten Sequenzen oder
AG.GGG.GAG.GTG.AGC.GCC.GAC.GAG.GAG.GGC.TTT. GAG.AAC.
aufweist.

2. Peptid, das das extrazelluläre Epitop der B Zellen-Membran-gebundenen Form von IgM odr IgG enthält, welches Epitop an der ausgeschiedenen Form des Immunglobulins nicht vorhanden ist, und das eine der folgenden Aminosäure-Sequenzen
EGEVS.ADEEG.FEN; oder
ELQLE.ESCAE.AQDGE.LDG
aufweist.

3. Peptid nach Anspruch 2, das an eine Substanz konjugiert ist, welche die Immunisierungsfähigkeit des Peptids erhöht.

4. Peptid nach Anspruch 3, bei dem die Substanz Hepatitis B-Oberflächenantigen,-Rumpfantigen oder Schlüsselloch-Napfschnecken-Hämocyanin ist.

5. Antikörper, der spezifisch an das extrazelluläre Epitop der B Zellen-Membran-gebundenen Form von IgM oder IgG bindet, welches Epitop an der ausgeschiedenen Form des Immunglobulins nicht vorhanden ist.

6. Antikörper nach Anspruch 5, der beim Binden die mit dem Membran-gebundenen Immunglobulin verknüpften B Zellen zur Zerstörung bezeichnet.

7. Antikörper nach Anspruch 5 oder 6, bei dem der Antikörper an ein Epitop auf einem der folgenden Peptid-Segmente
EGEVS.ADEEG.FEN;
ELQLE.ESCAE.AQDGE.LDG.
bindet.

8. Antikörper nach einem der Ansprüche 5 bis 7, bei dem der Antikörper ein monoklonaler Antikörper ist.

9. Antikörper nach Anspruch 8, bei dem der monoklonale Antikörper ein menschlicher monoklonaler Antikörper oder ein Fragment davon ist.

10. Fragment des Antikörpers nach einem der Ansprüche 5 bis 9 oder Peptid, das den Antigen-Bindungsteil des Fragments enthält, wobei das Fragment oder Peptid die Bindungsspezifität des Antikörpers besitzt.

11. Antikörper nach Anspruch 9, bei dem das menschliche Antikörperfragment ausgewählt ist aus der Gruppe, die besteht aus V_{H}, V_{L}, F_{V}, Fd, Fab, Fab', F(ab')₂ und Protein- oder Peptid-Analogen davon.

12. Antikörper nach Anspruch 8, bei dem der monoklonale Antikörper ein chimärer Antikörper mit tierischen variablen Bereichen und menschlichen konstanten Bereichen ist.

13. Antikörper nach Anspruch 12, bei dem der chimäre Antikörper bei Mäusen vorkommende variable Bereiche und menschliche konstante Bereiche hat.

14. Antikörper nach Anspruch 12 oder 13, bei dem der chimäre Antikörper den Antigen-Bindungsteil seines variablen Bereichs von einem Tier abgeleitet hat und andere Teile seines variablen Bereichs und seinen gesamten konstanten Bereich von einem Menschen abgeleitet hat.

15. Antikörper nach einem der Ansprüche 8 bis 9 oder 11 bis 14, bei dem der monoklonale Antikörper einer der folgenden Isotypen und Unterklassen ist: Mäuse-IgG2a, menschliches IgG1 und menschliches IgG3.

16. Beständige, stabile Zellinie, die den monoklonalen Antikörper nach einem der Ansprüche 8 bis 9 oder 11 bis 14 oder das Fragment nach Anspruch 10 erzeugt.

17. Zellinie nach Anspruch 16, die ein Hybridom ist.

18. Zellinie nach Anspruch 17, die ein Transfektom ist.

19. Monoklonaler anti-idiotyper Antikörper, der spezifisch ist für das Paratop des Antikörpers nach einem der Ansprüche 5 bis 15.

20. Monoklonaler anti-idiotyper Antikörper nach Anspruch 19, der ein chimärer Antikörper ist mit einem Antigen-Bindungsbereich tierischen Ursprungs und einem menschlichen konstanten Bereich oder einem Fragment davon.

21. Monoklonaler anti-idiotyper Antikörper nach Anspruch 20, bei dem das Antikörper-Fragment ausgewählt ist aus der Gruppe, die besteht aus V_{H}, V_{L}, F_{V}, Fd, Fab, Fab', F(ab')₂ und Protein- oder Peptid-Analogen davon.

22. Beständige stabile Zellinie, die den monoklonalen Antikörper nach einem der Ansprüche 19 bis 21 ausscheidet.

23. Pharmazeutische Zusammensetzung aufweisend einen Antikörper nach einem der Ansprüche 5 bis 15 oder 19 bis 21 oder das Peptid nach einem der Ansprüche 2 bis 4.

24. Pharmazeutische Zusammensetzung nach Anspruch 23 zur Behandlung einer Autoiminun-Krankheit, die im Zusammenhang steht mit einem speziellen Immunglobulin-Isotyp.

25. Pharmazeutische Zusammensetzung nach Anspruch 24, bei der das Immunglobulin IgG- oder IgM-Isotyp ist.

26. Pharmazeutische Zusammensetzung nach Anspruch 24 oder 25, bei der die Autoimmun-Krankheit rheumatische Arthritis, multiple Sklerose, Myasthenia gravis oder allgemeiner Lupus erythematodes ist.

27. Verwendung des Antikörpers nach einem der Ansprüche 5 bis 15 oder 19 bis 21 oder des Peptids nach einem der Ansprüche 2 bis 4 zur Herstellung einer pharmazeutischen Zusammensetzung zur Behandlung einer Autoimmun-Krankheit.

28. Verwendung nach Anspruch 27, bei der die Autoimmun-Krankheit rheumatische Arthritis, multiple Sklerose, Myasthenia gravis oder allgemeiner Lupus erythematodes ist.

29. Verwendung des Antikörpers nach einem der Ansprüche 5 bis 15 oder 19 bis 21 zur Bestimmung von Anzahlen und relativen Anteilen von spezielle Isotypen tragenden B Lymphozyten in gemischen Leukozyten-Populationen in einem Standardtest.

30. Verfahren zur Herstellung eines monoklonalen Antikörpers oder eines Fragments davon, welches Züchten der Zellinie nach den Ansprüchen 16 bis 18 oder 22 und Isolieren des erzeugten monoklonalen Antikörpers oder Fragments aufweist.

## Revendications

1. Séquence d'ADN qui code pour l'épitope extracellulaire de la forme de IgM ou IgG liée à la membrane de cellule B, ledit épitope n'étant pas présent sur la forme sécrétée desdites immunoglobulines, ladite séquence d'ADN comprenant l'une des séquences représentées par les lettres de cas supérieur de la figure 2A ou
AG.GGG.GAG.GTG.AGC.GCC.GAC.GAG.GAG.GGC.TTT.GAG.AAC.

2. Peptide contenant l'épitope extracellulaire de la forme de IgM ou IgG liée à la membrane de cellule B, ledit épitope n'étant pas présent sur la forme sécrétée des immunoglobulines et comprenant l'une des séquences d'acides aminés suivantes :
EGEVS.ADEEG.FEN; ou
ELQLE.ESCAE.AQDGE.LDG.

3. Peptide selon la revendication 2, conjugué à une substance qui accroît l'immunogénicité du peptide.

4. Peptide selon la revendication 3, dans lequel la substance est l'antigène d'enveloppe du virus de l'hépatite B, l'antigène nucléocapsidique ou l'hémocyanine de patelle.

5. Anticorps qui se fixe spécifiquement à l'épitope extracellulaire de la forme de IgM ou IgG liée à la membrane de cellule B, ledit épitope n'état pas présent sur la forme sécrétée desdites immunoglobulines.

6. Anticorps selon la revendication 5, qui, après fixation, marque les cellules B associées à l'immunoglobuline liée à la membrane pour une destruction.

7. Anticorps selon la revendication 5 ou 6, dans lequel ledit anticorps se fixe à un épitope sur l'un des segments peptidiques suivants :
EGEVS.ADEEG.FEN;
ELQLE.ESCAE.AQDGE.LDG.

8. Anticorps selon l'une quelconque des revendications 5 à 7, dans lequel ledit anticorps est un anticorps monoclonal.

9. Anticorps selon la revendication 8, dans lequel ledit anticorps monoclonal est un anticorps monoclonal humain ou un de ses fragments.

10. Fragment de l'anticorps selon l'une quelconque des revendications 5 à 9, ou bien peptide contenant la portion de liaison à l'antigène dudit fragment, ledit fragment ou ledit peptide présentant la spécificité de liaison dudit anticorps.

11. Anticorps selon la revendication 9, dans lequel le fragment d'anticorps humain est choisi dans le groupe constitué de V_{H}, V_{L}, F_{V}, Fd, Fab, Fab', F(ab')₂ et protéine ou analogues peptidiques.

12. Anticorps selon la revendication 8, dans lequel ledit anticorps monoclonal est un anticorps chimère présentant des régions variables animales et des régions constantes humaines.

13. Anticorps selon la revendication 12, dans lequel ledit anticorps chimère présente des régions variables murines et des régions constantes humaines.

14. Anticorps selon la revendication 12 ou 13, dans lequel ledit anticorps chimère présente la portion de liaison à l'antigène de sa région variable provenant d'un animal ou d'autres portions de sa région variable et sa région constante totale provenant d'un homme.

15. Anticorps selon l'une quelconque des revendications 8 et 9 ou 11 à 14, dans lequel ledit anticorps monoclonal est l'un des isotypes et sous-classes suivants : IgG2a murin, IgG1 humain et IgG3 humain.

16. Lignée cellulaire stable ininterrompue qui produit l'anticorps monoclonal selon l'une quelconque des revendications 8 à 9 ou 11 à 14 ou le fragment selon la revendication 10.

17. Lignée cellulaire selon la revendication 16 qui est un hybridome.

18. Lignée cellulaire selon la revendication 17 qui est un transfectome.

19. Anticorps monoclonal anti-idiotype spécifique du paratope de l'anticorps selon l'une quelconque des revendications 5 à 15.

20. Anticorps monoclonal anti-idiotype selon la revendication 19 qui est un anticorps chimère ayant une région de liaison à l'antigène d'origine animale et une région constante humaine ou un de ses fragments.

21. Anticorps monoclonal anti-idiotype selon la revendication 20, dans lequel le fragment d'anticorps est choisi dans le groupe constitué de V_{H}, V_{L}, F_{V}, Fd, Fab, Fab', F(ab')₂ et protéine ou analogues peptidiques.

22. Lignée cellulaire stable ininterrompue qui sécrète l'anticorps monoclonal selon l'une quelconque des revendications 19 à 21.

23. Composition pharmaceutique comprenant un anticorps selon l'une quelconque des revendications 5 à 15 ou 19 à 21, ou bien un peptide selon l'une quelconque des revendications 2 à 4.

24. Composition pharmaceutique selon la revendication 23, destinée au traitement d'une maladie auto-immunitaire associée à un isotype d'immunoglobuline particulier.

25. Composition pharmaceutique selon la revendication 24, dans laquelle l'immunoglobuline est l'isotype de IgG ou IgM.

26. Composition pharmaceutique selon la revendication 24 ou 25, dans laquelle la maladie auto-immunitaire est la polyarthrite rhumatoïde, la sclérose en plaque, la myasthénie grave ou le lupus érythémateux aigu disséminé.

27. Utilisation de l'anticorps selon l'une quelconque des revendications 5 à 15 ou 19 à 21, ou du peptide selon l'une quelconque des revendications 2 à 4 pour la préparation d'une composition pharmaceutique destinée au traitement d'une maladie auto-immunitaire.

28. Utilisation selon la revendication 27, dans laquelle la maladie auto-immunitaire est la polyarthrite rhumatoïde, la sclérose en plaque, la myasthénie grave ou le lupus érythémateux aigu disséminé.

29. Utilisation de l'anticorps selon l'une quelconque des revendications 5 à 15 ou 19 à 21 pour la détermination, dans un dosage standard, du nombre et des proportions relatives des lymphocytes B portant les isotypes particuliers dans des populations mixtes de leucocyte.

30. Procédé de production d'un anticorps monoclonal ou d'un de ses fragments, lequel comprend la culture de la lignée cellulaire selon les revendications 16 à 18 ou 22 et l'isolement de l'anticorps monoclonal ou du fragment produits.
